# EUROPEAN PATENT SPECIFICATION

(11) **EP 1 011 880 B1**
(45) Date of publication and mention of the grant of the patent: **10.07.2002**
(21) Application number: 97944961.8
(22) Date of filing: 08.10.1997
(51) Int. Cl.: B08B 9/04, C12M 1/00, F16L 55/38, F16L 55/40, F28G 1/12

(54) **Photobioreaktor having mobile cleaning means**
Photobioreaktor mit beweglichen Reinigungsmittel
Photbioreacteur avec moyens de nettoyage mobiles

(30) Priority: 10.10.1996 GB 9621130
(43) Date of publication of application: 28.06.2000
(73) Proprietor: Biofence Limited, Port Talbot SA12 6HZ (GB)
(72) Inventor: JENKINS, David, Paul +di, - (GB)
(74) Representative: Davies, Gregory Mark
(86) International application number: GB9702757
(87) International publication number: WO9815362

(56) References cited:
- DE-U- 29 607 285
- FR-A- 2 576 034
- FR-A- 2 697 027
- US-A- 4 569 097
- US-A- 5 176 204

## Description

The present invention relates to apparatus for cleaning at least one conduit of a photobioreactor.

A common problem when fluid flows through a conduit is that there is an accumulation of debris on the internal walls. This is a particular problem with photobioreactors.

Typically, photobioreactors comprise substantially optically transparent tubes in which photosynthetic micro-organisms, such as algae or the like, dispersed within a culture medium in such tubes, can utilise light to undergo photosynthetic reactions.

Generally, after a period of time in the photobioreactor, micro-organisms accumulate on the internal walls of the tube to which they adhere. Adhesion of the micro-organisms to the internal tube walls leads to a reduction in the volume of culture within the tube which may be exposed to light, and consequently to less efficient biomass production. It is therefore desirable for a photobioreactor system to incorporate a cleaning mechanism which prevents adhesion of the micro-organisms to the internal walls of the tube. It is desirable for any such cleaning mechanism to be automatic in order to optimise biomass production by obviating the need to shut the reactor down.

Several cleaning systems have been previously proposed for photobioreactors. An example of such a cleaning system comprises pockets at each end of the photobioreactor tube. Periodic reversal of the flow of liquid culture medium in the tube instigates movement of this cleaning element from one pocket to the other. As it moves the cleaning element contacts the internal surface of the tube and removes any micro-organisms which are adhering thereto. Such pocket-type cleaning systems can be expensive to use because they require interruption of normal operation and thereby increase the production costs of the photobioreactor.

US 4,569,097 discloses of a plurality of tube cleaning elements of various shapes, such as cubic, spherical and cylindrical, which continuously circulate around a cleaning loop for a heat exchanger. However, these are of a size which substantially fills the interior diameter of the tubes being cleaned so as to rub and effectively clean their inner surface upon repeated passage therethrough. Such large cleaning elements would not be suitable for use in a photobioreactor where there must be room for algae to circulate and even partial blockage of the passageway may lead to an undesirable build-up of oxygen.

FR 2,576,034 discloses use of a plurality of substantially spherical balls for cleaning the interior walls of a photobioreactor. These balls are made of an elastic polymer or of rubber. They circulate along with algae and culture medium and are said to clean the walls by rubbing thereagainst. The diameter of these balls appears to be greater than 50% the diameter of the tube in which they circulate, and it is believed that they are not particularly effective.

It is the object of the present invention to provide a substantially improved and more cost effective apparatus for cleaning internal walls of photobioreactor tubes.

The invention provides photobioreactor apparatus comprising at least one tubular conduit, mobile cleaning means within said conduit; at least one inlet connectable to said conduit for introducing culture medium into the conduit in such a manner as to propel said cleaning means along said conduit, said conduit having a plurality of apertures arranged to permit part of the culture medium to pass from said conduit into a collection vessel and to prevent passage of said cleaning means thereto, and pump. means for pumping culture medium passing through said apertures to said inlet; characterised in that said cleaning means comprise a plurality of granules or beads which are substantially cylindrical in shape and have an average major dimension of 1 to 3 mm and which can be passed within the culture medium along the conduit.

Thus, the culture medium may be pumped under pressure into a respective tube from the collection vessel, the culture medium then being recycled to the photobioreactor tube, while the cleaning means are maintained within the closed photobioreactor circuit. New culture medium may advantageously be introduced into the collection vessel when required so as to replenish depleted nutrients in the culture medium.

Preferably the inlet is provided substantially downstream relative to flow of the culture medium from the or each aperture. Thus, advantageously, when the culture medium is pumped back into the respective tube from the collection vessel, the medium is under pressure and induces a flow in the medium already present in the photobioreactor tube downstream of the apertures. The flow of the culture medium between the inlet and the respective aperture(s) is substantially reduced by the flow of a substantial portion of the culture medium into the collection vessel. The flow of culture medium downstream of the respective aperture(s) advantageously induces an increase in the flow rate in the culture medium in the tube between the apertures and the inlet, which increased flow advantageously allows the cleaning means to pass along the photobioreactor tube containing the apertures.

Typically, the inlet is arranged to introduce the culture medium substantially adjacent the conduit.

In a preferred embodiment of the invention, approximately 40-70% of a culture medium may be passed through the aperture or apertures to the collection vessel. Preferably, the collection vessel is fitted with a gas valve, which advantageously allows gaseous by-products of photosynthesis which have accumulated in the culture medium to be released therefrom. Therefore, the apparatus has the further advantage of permitting removal of gaseous waste by-products of photosynthesis, such as oxygen, from the medium, in addition to permitting cleaning of the photobioreactor tube. The de-gassed culture medium may then be re-introduced into the photobioreactor tube by a pump mechanism as described above.

According to the invention, the cleaning means comprises a plurality of granules or beads which can be passed within the culture medium along the conduit. Such granules or beads clean the internal walls of the conduit by contacting the internal walls as they are dispersed in the flowing culture medium.

In a preferred embodiment of the invention the beads or granules are propelled around the photobioreactor circuit directly by a pump mechanism through which the beads or granules pass. Preferably a centrifugal pump is used, and where such a pump is used, the algae used in the culture medium may have relatively thick cell walls.

Preferably the beads or granules are made of polystyrene, polypropylene, nylon, PVC or polyvinylacetate, and the conduit comprises an acrylic plastics material, unplasticised PVC or glass.

The photobioreactor apparatus according to the present invention is preferably used for culturing micro-organisms. Thus a further aspect of the present invention is a method of culturing micro-organisms using the photobioreactor apparatus according to the present invention. The method preferably comprises introducing culture medium into the conduit of the photobioreactor via the inlet in such a manner as to propel the cleaning means along the conduit, the cleaning means generally passing within the culture medium along the conduit. Part of the culture medium which has passed from the conduit via the plurality of apertures into the collection vessel, is then preferably collected and the cleaning means are desirably substantially prevented by the plurality of apertures from passing into the collection vessel. The culture medium passing through the plurality of apertures is then preferably pumped to the inlet by pump means.

The method preferably further comprises releasing accumulated gaseous products of photosynthesis by a gas valve provided in the collection vessel. Thus the method has the dual advantage of not only cleaning the tubes but permitting removal of gaseous products of photosynthesis.

## Claims

1. Photobioreactor apparatus comprising:
at least one tubular conduit;
mobile cleaning means within said conduit;
at least one inlet connectable to said conduit for introducing culture medium into the conduit in such a manner as to propel said cleaning means along said conduit, said conduit having a plurality of apertures arranged to permit part of the culture medium to pass from said conduit into a collection vessel and to prevent passage of said cleaning means thereto; and
pump means for pumping culture medium passing through said apertures to said inlet;
**characterised in that** said cleaning means comprise a plurality of granules or beads which are substantially cylindrical in shape and have an average major dimension of 1 to 3 mm and which can be passed within the culture medium along the conduit.

2. Apparatus according to claim 1, wherein said inlet is provided substantially downstream relative to flow of culture medium from said apertures.

3. Apparatus according to claim 1 or 2, wherein said inlet is arranged so as to introduce said culture medium substantially adjacent the at least one conduit.

4. Apparatus according to any of claims 1 to 3, wherein said collection vessel has a gas valve.

5. Apparatus according to any of claims 1 to 4, wherein said culture medium is propelled along said conduit directly by a pump mechanism which in turn propels the beads or granules along the conduit.

6. Apparatus according to any of claims 1 to 5, wherein said beads are of polystyrene, polypropylene, nylon, PVC or polyvinylacetate.

7. Apparatus according to any of claims 1 to 6, wherein said conduit comprises an acrylic plastics material, unplasticised PVC or glass.

8. Use of a photobioreactor apparatus according to any preceding claim for culturing micro-organisms.

9. A method of culturing micro-organisms in a photobioreactor apparatus according to any of claims 1 to 7, said method comprising:
(a) introducing culture medium into said conduit via said inlet in such a manner as to propel said cleaning means along said conduit, said cleaning means being passed within the culture medium along the conduit;
(b) collecting part of the culture medium which has passed from said conduit via said plurality of apertures into said collection vessel, wherein said cleaning means are substantially prevented by said plurality of apertures from passing from said conduit into said collection vessel; and
(c) pumping said culture medium passing through said apertures to said inlet by said pump means.

10. A method according to claim 9, wherein accumulated gaseous products of photosynthesis within the photobioreactor apparatus are released by a gas valve in said collection vessel.

## Patentansprüche

1. Photobioreaktor enthaltend:
mindestens eine rohrförmige Leitung;
bewegliche Reinigungsmittel innerhalb der Leitung;
mindestens einen mit der Leitung verbindbaren Einlaß, um derart Kulturmedium in die Leitung einzuführen, daß die Reinigungsmittel längs der Leitung vorwärts getrieben werden, eine Anzahl von Öffnungen in der Leitung in solcher Anordnung, daß ein Teil des Kulturmediums aus der Leitung in einen Sammelbehälter eintreten kann und
eine Passage der Reinigungsmittel dahinein verhindert ist; und Pumpmittel, um das durch die Öffnungen durchgeflossene Kulturmedium in den Einlaß zu pumpen, **dadurch gekennzeichnet, daß** die Reinigungsmittel eine Anzahl von Granulen oder Körnern umfassen, die im wesentlichen eine zylindrische Form besitzen und eine durchschnittliche Hauptdimension von 1 - 3 mm aufweisen und innerhalb des Kulturmediums entlang der Leitung strömen können.

2. Gerät nach Anspruch 1, worin der Einlaß im wesentlichen stromab relativ zum Fluß des Kulturmediums aus den Öffnungen angeordnet ist.

3. Gerät nach Anspruch 1 oder 2, worin der Einlaß so angeordnet ist, daß das Kulturmedium im wesentlichen die mindestens eine Leitung berührend eingeführt wird.

4. Gerät nach einem der Ansprüche 1 bis 3, worin das Sammelgefäß ein Gasventil besitzt.

5. Gerät nach einem der Ansprüche 1 bis 4, worin das Kulturmedium entlang der Leitung vorwärts getrieben wird durch einen Pumpmechanismus der seinerseits die Körner oder Granulen entlang der Leitung vorwärts treibt.

6. Gerät nach einem der Ansprüche 1 bis 5, worin die Körner aus Polystyrol, Polypropylen, Nylon, PVC oder Polyvinylacetat bestehen.

7. Gerät nach einem der Ansprüche 1 - 6, worin die Leitung ein akrylisches Kunststoffmaterial, nicht-weichgemachtes PVC oder Glas umfaßt.

8. Verwendung eines Photobioreaktors gemäß einem der vorhergehenden Ansprüche zur Kultur von Mikroorganismen.

9. Verfahren zum Kultivieren von Mikroorganismen in einem Photobioreaktor nach einem der vorhergehenden Ansprüche, umfassend:
(a) Einführen des Kulturmediums über den Einlaß in die Leitung in solcher Weise, daß die Reinigungsmittel entlang der Leitung vorwärts getrieben werden, wobei die Reinigungsmittel innerhalb des Kulturmediums entlang der Leitung geströmt werden;
(b) Sammeln eines Teils des Kulturmediums, das aus der Leitung über die Öffnung in den Sammelbehälter geströmt ist, wobei die Reinigungsmittel im wesentlichen durch die Anzahl von Öffnungen daran gehindert sind, aus der Leitung in das Sammelgefäß zu strömen; und
(c) Pumpen des durch die Öffnungen geströmten Kulturmediums mittels des Pumpmittels in den Einlaß.

10. Verfahren nach Anspruch 9, worin gasförmige Produkte, die sich während der Photosynthese innerhalb des Photobioreaktors angesammelt haben, durch ein Gasventil im Sammelbehälter freigegeben werden.

## Revendications

1. Dispositif photobioréacteur, comprenant:
au moins un conduit tubulaire ;
des moyens de nettoyage mobiles dans ledit conduit ;
au moins une entrée pouvant être raccordée audit conduit pour introduire un milieu de culture dans le conduit, de manière à entraîner lesdits moyens de nettoyage le long dudit conduit, ledit conduit ayant une pluralité d'ouvertures disposées pour permettre, à une partie du milieu de culture de passer dudit conduit dans un vase de collecte et pour empêcher le passage desdits moyens de nettoyage vers celui-ci ; et
des moyens de pompage pour pomper le milieu de culture passant à travers lesdites ouvertures vers ladite entrée ;
**caractérisé en ce que** lesdits moyens de nettoyage comprennent une pluralité de granulés ou de billes qui sont de forme sensiblement cylindrique et ont une dimension principale moyenne de 1 à 3 mm, et qui peuvent être envoyés dans le milieu de culture le long du conduit.

2. Dispositif selon la revendication 1, dans lequel ladite entrée est prévue sensiblement en aval par rapport à l'écoulement du milieu de culture depuis lesdites ouvertures.

3. Dispositif selon la revendication 1 ou 2, dans lequel ladite entrée est placée de manière à introduire ledit milieu de culture de manière sensiblement adjacente au conduit au nombre d'au moins un.

4. Dispositif selon l'une quelconque des revendications 1 à 3, dans lequel ledit vase de collecte a une vanne à gaz.

5. Dispositif selon l'une quelconque des revendications 1 à 4, dans lequel ledit milieu de culture est entraîné le long dudit conduit directement par un mécanisme à pompe qui, à son tour, entraîne les billes ou les granulés le long du conduit.

6. Dispositif selon l'une quelconque des revendications 1 à 5, dans lequel lesdites billes sont en polystyrène, polypropylène, nylon, PVC ou polyvinylacétate.

7. Dispositif selon l'une quelconque des revendications 1 à 6, dans lequel ledit conduit comprend une matière plastique acrylique, du PVC non plastifié ou du verre.

8. Utilisation d'un dispositif photobioréacteur selon l'une quelconque des revendications précédentes pour cultiver des microorganismes.

9. Procédé de culture de microorganismes dans un dispositif photobioréacteur selon l'une quelconque des revendications 1 à 7, ledit procédé comprenant les étapes consistant à :
(a) introduire un milieu de culture dans ledit conduit via ladite entrée de manière à entraîner lesdits moyens de nettoyage le long dudit conduit, lesdits moyens de nettoyage étant passé dans le milieu de culture le long dudit conduit ;
(b) récupérer la partie du milieu de culture qui est passée dudit conduit via ladite pluralité d'ouverture dans ledit vase de collecte, dans lequel lesdits moyens de nettoyage sont sensiblement empêchés par ladite pluralité d'ouvertures de passer dudit conduit dans ledit vase de collecte ; et
(c) pomper ledit milieu de culture passant à travers lesdites ouvertures vers ladite entrée par lesdits moyens de pompage.

10. Procédé selon la revendication 9, dans lequel les produits gazeux de la photosynthèse accumulés dans le dispositif photobioréacteur sont évacués par une vanne à gaz dans ledit vase de collecte.
